# EUROPÄISCHE PATENTSCHRIFT

(11) **EP 0 756 605 B1**
(45) Veröffentlichungstag und Bekanntmachung des Hinweises auf die Patenterteilung: **11.08.1999**
(21) Anmeldenummer: 95918582.8
(22) Anmeldetag: 21.04.1995
(51) Int. Cl.: C07K 14/74, C07K 14/47, C07K 7/08, A61K 38/10, A61K 38/17

(54) **PEPTIDE ALS THERAPEUTIKUM FÜR AUTOIMMUNERKRANKUNGEN**
PEPTIDES AS THERAPEUTIC AGENTS FOR THE TREATMENT OF AUTO-IMMUNE DISEASES
PEPTIDES UTILISE COMME AGENTS THERAPEUTIQUES DANS LE TRAITEMENT DE MALADIES AUTO-IMMUNES

(30) Priorität: 21.04.1994 DE 4413938
(43) Veröffentlichungstag der Anmeldung: 05.02.1997
(73) Patentinhaber: Wildner, Gerhild, 80636 München (DE); Thurau, Stephan, 80331 München (DE)
(72) Erfinder: Wildner, Gerhild, 80636 München (DE); Thurau, Stephan, 80331 München (DE)
(74) Vertreter: Wibbelmann, Jobst, Dr., Dipl.-Chem.
(86) Internationale Anmeldenummer: EP9501512
(87) Internationale Veröffentlichungsnummer: WO9529194

(56) Entgegenhaltungen:
- WO-A-94/04171
- WO-A-94/04557
- J.AUTOIMMUNITY, Bd. 4, Nr. 3, Juni 1991 Seiten 507-516, S.R.THURAU U.A. 'Induction of oral tolerance to S-antigen induced experimental autoimmune uveitis by a uveitogenic 20mer peptide' in der Anmeldung erwähnt
- EUR.J.IMMUNOL., Bd. 24, Nr. 11, 1994 Seiten 2579-2585, G.WILDNER U.A. 'Cross-reactivity between an HLA-B27-derived peptide and a retinal autoantigen peptide...'

## Beschreibung

Autoimmunerkrankungen (Rheuma, Multiple Sklerose, Colitis Ulcerosa, Autoimmun-Uveitis u.v.a) sind Erkrankungen, bei denen das körpereigene Abwehrsystem (Immunsystem) Strukturen des eigenen Organismus nicht mehr toleriert, sondern wie einen eingedrungenen Erreger attackiert. Dies hat zur Folge, daß körpereigenes Gewebe geschädigt oder sogar zerstört wird und dadurch Schmerzen und körperliche Ausfallserscheinungen auftreten (z.B. steife Gelenke bei Rheuma; allgemeine Bewegungsschwäche, Inkontinenz, Sprachstörungen bei Multipler Sklerose; schwere Durchfälle, Ernährungsstörungen bis hin zur Darmperforation mit tödlichem Ausgang bei Colitis Ulcerosa; bei Uveitis Verminderung der Sehstärke, die bis zur Erblindung führen kann).

Die Uveitis ist eine Entzündung im Inneren des Auges, die Regenbogenhaut, Strahlenkörper, Glaskörper, Netz- und/oder Aderhaut betrifft. Die Entzündung verläuft bei betroffener Regenbogenhaut oft schmerzhaft und kann zur Blockade der Pupillenreaktion durch Verkleben der Regenbogenhaut an der Vorderseite mit der Hornhaut und/oder an der Rückseite mit der Linse führen. Zelluläre Beschläge an der Innenseite der Hornhaut und auf der Linse sowie zelluläre Einwanderungen in die Vorderkammer und in den Glaskörper des Auges, manchmal gefolgt vom Wachstum schleierartiger Membranen, die sich wie Vorhänge durch den Glaskörper ziehen oder auf die Netzhaut legen, beeinträchtigen das Sehvermögen. Entzündungen von Netz- und Aderhaut zeigen sich in Ödemen, zellulären Infiltraten, später in atrophischen Veränderungen. Nicht selten sind die Netzhautgefäße an diesen Entzündungen beteiligt. Ödematöse Schwellung des Sehnervenkopfes kann zusätzlich zur Störung der neuronalen Verarbeitung von Lichtreizen führen. Während die oben beschriebenen Phänomene teilweise reversibel sind, stellt eine Zerstörung der Struktur der Netzhaut ein irreversibles Ereignis dar, das eine bleibende Sehverminderung bis zur Erblindung mit sich führt.

Die oben beschriebenen uveitischen Symptome können auch Begleiterscheinungen bei anderen Autoimmunerkrankungen sein, wie z.B. Erkrankungen des rheumatischen Formenkreises oder auch parainfektiös auftreten. Diese Entzündung wird vom Immunsystem initiiert, im weiteren Verlauf sind auch andere Zelltypen an der Entzündungsreaktion beteiligt.

Autoimmunerkrankungen verlaufen in fast allen Fällen chronisch, entweder schubweise wiederkehrend oder permanent schleichend. Die durch das Immunsystem zerstörten Gewebe können in den seltensten Fällen regeneriert werden. Wenn, wie bei der Multiplen Sklerose oder der Uveitis, Nervengewebe betroffen ist, ist eine Regeneration überhaupt nicht möglich. Die Ursache für die Entstehung solcher Autoimmunerkrankungen sind noch weitgehend unbekannt, in manchen Fällen vermutet man Infektionen, durch die das Immunsystem fehlgeleitet wird.

Die konventionellen Therapiemaßnahmen, die im Falle von Autoimmunerkrankungen eingeleitet werden, betreffen nicht nur den selbstreaktiven ("autoagressiven") Teil des Immunsystems, sondern supprimieren generell immunreaktive Zellen, die eigentlich zur Abwehr von eingedrungenen Erregern oder von Tumorzellen dienen sollten. Neben einer verringerten allgemeinen Immunabwehr und einer möglichen Entstehung von Tumoren haben die konventionellen Immunsuppressiva wie Corticosteroide, Cyclosporin A und Zytostatika auch viele nicht-immunologische und z.T. lebensbedrohliche Nebenwirkungen zur Folge.

Für viele (Autoimmun-)Erkrankungen wurde eine erbliche Disposition aufgrund statistischer Korrelation der Krankheit mit bestimmten Histokompatibilitäts-Antigenen (HLA, ursprünglich als Transplantationsantigene entdeckt) vermutet. Als Mechanismus wurde eine Verwechslung von Erreger-Antigenen (Bakterien, Viren) mit körpereigenen HLA-Antigenen durch das Immunsystem postuliert.

In den letzten Jahren wurden viele Versuche unternommen, regulierend in das Immunsystem einzugreifen, um das normale Gleichgewicht zwischen Selbsttoleranz und Abwehr von Krankheitserregern wieder herzustellen. Unter anderem versucht man, über "immunprivilegierte" Bereiche des Organismus eine immunologische Toleranz zu induzieren. Zu den immunprivilegierten Bereichen gehört z.B. auch der Darm der Säugetiere, über den man Toleranz ("orale Toleranz") gegen Proteinmoleküle ("Antigene") induzieren kann, ein Mechanismus, der vermutlich verhindern soll, daß ständig eine Immunreaktion gegen die aufgenommene Nahrung erzeugt wird. Die orale Verabreichung von "Autoantigenen" (körpereigene Proteine, gegen die im Falle von Autoimmunerkrankungen die Aggression des Immunsystems gerichtet ist), kann folglich Toleranz, d.h. Nicht-Reagieren auf körpereigenes Gewebe induzieren, selbst wenn der Ort der Autoaggression, nämlich das betroffene Gewebe, örtlich weit vom Darm entfernt ist. Die orale Toleranzinduktion ist eine Form der Immunsuppression, die hoch spezifisch ist, d.h. nur die Immunreaktion auf das betroffene Antigen betrifft. Es sind daher keine Nebenwirkungen wie bei einer allgemeinen Immunsuppression zu erwarten. Unerwünschte pharmakologisch-toxikologische Wirkungen sind bisher bei der oralen Verabreichung von Autoantigenen nicht beobachtet worden und auch nicht zu erwarten.

Für einige Autoimmunerkrankungen sind die Autoantigene bekannt und können daher für spezifische Therapien eingesetzt werden. In manchen Fällen gibt es nur ein Antigen, gegen das die Immunreaktion gerichtet ist (Collagen II bei Rheuma, Insulin bei Diabetes), im Fall der Autoimmunuveitis kennt man zwei Proteine aus der Photorezeptorschicht im Auge, die im Tiermodell Uveitis auslösen können und gegen die bei Uveitispatienten eine Immunreaktion nachweisbar ist.

Dies sind das retinale S-Antigen und das interphotorezeptorretinolbindende Protein (IRBP). Darüberhinaus gibt es innerhalb dieser Proteine bestimmte Abschnitte (Epitope), die in Form von Peptiden verabreicht, ebenfalls eine Uveitis induzieren können. Eine so induzierte experimentelle Uveitis läßt sich verhindern, wenn S-Antigen, IRBP oder bestimmte davon abgeleitete Peptide oral verabreicht werden (orale Toleranz) (siehe u.a. Thurau, S.R., Chan, C.-C., Suh, E., Nussenblatt, R. B.; Induction of oral tolerance to S-Antigen induced experimental autoimmune uveitis by a uveitogenic 20mer peptide. J. Autoimmun. **4**, 507-516 (1991)).

Aus der Arbeit von G. Wildner et al, Eur. J. Immunol., Band 24, Nr. 11, 1994 Seiten 2579 - 2585 ist bekannt, daß die orale Verabreichung bestimmter HLA-Peptide eine durch das S-Antigen induzierte Erkrankung unterdrücken kann.

In der WO-A-9404171 werden ausdrücklich die folgenden Peptide genannt. Unter anderem wird dort behauptet, daß diese Peptide allgemein zur Behandlung von Autoimmunerkrankungen eingesetzt werden können:

Leu-Arg-Ser-Trp-Thr-Ala-Ala-Asp-Thr-Ala-Ala-Gln-Ile-Thr-
Gln-Arg-Lys-Trp-Glu-Ala-Ala;

Asp-Leu-Arg-Ser-Trp-Thr-Ala-Ala-Asp-Thr-Ala-Ala-Gln-Ile-
Thr-Gln-Arg;

Asp-Leu-Arg-Ser-Trp-Thr-Ala-Ala-Asp-Thr-Ala-Ala-Gln-Ile-
Thr-Gln;

Asp-Leu-Arg-Ser-Trp-Thr-Ala-Ala-Asp-Thr-Ala-Ala-Gln-Ile-
Thr;

Asp-Leu-Ser-Ser-Trp-Thr-Ala-Ala-Asp-Thr-Ala-Ala-Gln-Ile-
Thr-Gln-Arg;

Asp-Leu-Ser-Ser-Trp-Thr-Ala-Ala-Asp-Thr-Ala-Ala-Gln-Ile-
Thr-Gln-Arg-Lys-Trp-Glu;

Asp-Leu-Arg-Ser-Trp-Thr-Ala-Ala-Asp-Thr-Ala-Ala-Gln-Ile-
Thr-Gln-Arg-Lys-Trp;

Leu-Arg-Ser-Trp-Thr-Ala-Ala-Asp-Thr-Ala-Ala-Gln-Ile-Thr-
Gln-Arg-Lys-Trp;

Asp-Leu-Ser-Ser-Trp-Thr-Ala-Ala-Asp-Thr-Ala-Ala-Gln-Ile-
Thr-Gln-Arg-Lys-Trp-Glu-Ala-Ala;

Asp-Leu-Ser-Ser-Trp-Thr-Ala-Ala-Asp-Thr-Ala-Ala-Gln-Ile-
Thr-Gln-Arg-Lys.

Die Bereitstellung von Antigenproteinen ist nicht nur mit beträchtlichen Schwierigkeiten verbunden. Darüberhinaus haben sie eine Vielzahl von Nachteilen:
1) Das Antigen muß im Allgemeinen aus natürlichem Gewebe isoliert werden. Hierbei kann es unter Umständen zu einer Infektion (Viren, Viroide oder Bakterien) bzw. Transfektion mit (retro)viraler oder bakterieller DNA kommen.
2) Das Antigen kann allenfalls mit großen Aufwand "gentechnologisch" synthetisiert werden.
3) Die Lagerung von Proteinen, wie Antigenproteinen, über längere Zeit kann im Einzelfall Schwierigkeiten bereiten. Im ungünstigen Falle kann auch bei aufwendigen Lagerbedingungen die Stabilität, Aktivität bzw. Wirksamkeit eines Proteins nur für eine kurze Zeitdauer gewährleistet werden.
4) Die Gefahr einer Allergisierung im Sinne einer Lebensmittel- oder Kontaktallergie ist bei Antigenproteinen erhöht, da es leicht zu einer Vernetzungswirkung auf Zelloberflächen kommt. Dies ist besonders bei Patienten mit Autoimmunerkrankungen wichtig, da sich unter ihnen eine deutlich erhöhte Zahl von Allergikern findet.

Der Erfindung liegt das Problem zugrunde, hier Abhilfe zu schaffen.

Dieses Problem wird durch ein Peptid, wie es in den Ansprüchen definiert wird, gelöst.

Die Erfindung bezieht sich auf die Verwendung eines Peptids aus 7 bis 30 Aminosäuren mit der Sequenz A-Leu-B-Ser-C-Thr-Ala-Ala-D, wobei B und C je für eine Aminosäure
und A und D je für monomere oder polymere organische Endgruppen stehen zur Herstellung eines Mittels zur oralen Verabreichung bei der Behandlung von Autoimmunerkrankungen; ausgenommen ein Peptid (B7PD), das die Sequenz Ala-Leu-Asn-Glu-Asp-Leu-Arg-Ser-Trp-Thr-Ala-Ala-Asp-Thr umfaßt.

Es wurde desweiteren gefunden, daß ein Peptid mit einer gegenüber der vorstehend angegebenen Kernsequenz -Leu-B-Ser-C-Thr-Ala-Ala- carboxyterminal um zwei Aminosäuren, Asparaginsäure und Glutaminsäure, verlängerten Kernsequenz ebenfalls hervorragende therapeutische Eigenschaften aufweist.

Gegenstand der Erfindung ist weiterhin ein Peptid aus 9 bis 30 Aminosäuren mit der Sequenz A-Leu-B-Ser-C-Thr-Ala-Ala-Asp-Glu-D, wobei B und C jeweils für eine Aminosäure
stehen und A und D für monomere oder polymere organische Endgruppen stehen.

Es hat sich im Zuge der Untersuchungen gezeigt, daß die Bindung der erfindungsgemäßen Peptide an den T-Zell-Rezeptor und HLA (humanes Leukozytenantigen) über mehrere Aminosäuren innerhalb einer Kernsequenz diskontinuierlich verteilt ist. Zwischen diesen für die Bindung essentiellen Aminosäuren gibt es Positionen, die nicht an der Bindung teilnehmen und daher ausgetauscht werden können. Entscheidend für die Funktion scheint somit nicht eine durchgehende Sequenz von Aminosäuren zu sein, sondern einzelne Positionen innerhalb des Peptids, die mit dem T-Zell-Rezeptor und dem HLA-Antigen gleichzeitig wechselwirken.

Dementsprechend sind vor und hinter der Kernsequenz mit 7 bzw. 9 Aminosäuren (Position A bzw. Position D) sowie innerhalb der Kernsequenz (Positionen B und C) Aminosäureaustausche ohne wesentliche biologisch-funktionelle Einbußen möglich.

Die variablen Positionen B und C liegen innerhalb der 7 bzw. 9 Aminosäuren langen Kernsequenz und stehen für eine oder mehrere Aminosäuren. Da die sterischen Gegebenheiten innerhalb der Kernsequenz für die biologisch relevante Wechselwirkung einzelner Positionen innerhalb der Kernsequenz mit dem HLA-Molekül und dem T-Zell-Rezeptor von entscheidender Bedeutung sind, sind hier Variationen nur in einem relativ begrenzten Rahmen möglich. Es hat sich im Rahmen der Untersuchungen gezeigt, daß B und C bevorzugt für je eine einzelne Aminosäure stehen.

Insbesondere betrifft die Erfindung Peptide nach den vorstehend genannten Maßgaben, in welchen B für Serin oder Threonin steht. Die Austauschbarkeit der Aminosäuren an Position B der Kernsequenzen wurde experimentell wiederholt nachgewiesen. Es hat sich jedoch im Zuge der Untersuchungen gezeigt, daß bei einer Substitution der Gruppe B durch Arginin keine therapeutisch wirksamen Peptide erhalten werden.

In einer bevorzugten Form des erfindungsgemäßen Peptids bedeutet ferner C Threonin, Tryptophan, Glutaminsäure oder Serin. Auch hier können andere Aminosäuren diese Position in den Kernsequenzen einnehmen, ohne die therapeutische Wirksamkeit wesentlich zu beeinflussen. Somit scheint die Aminosäure an Position C der erfindungsgemäßen Peptide lediglich von untergeordneter Bedeutung für die Bindung innerhalb des HLA-Peptid-T-Zell-Rezeptor-Komplexes zu sein.

Die hinsichtlich der Kernsequenz amino- und carboxyterminalen Endgruppen A und D befinden sich bei einer Integration der erfindungsgemäßen Peptide im HLA-Peptid-T-Zell-Rezeptor-Komplex offenbar außerhalb der Bindungsstelle und haben dementsprechend kaum noch eine biologisch-funktionelle Bedeutung. Daher können an diesen Positionen eine Vielzahl von Gruppen mit unterschiedlicher Länge und unterschiedlicher Sperrigkeit vorliegen, ohne die therapeutische Wirkung der Peptide bedeutend zu beeinflussen.

Die monomeren oder polymeren organischen Endgruppen A und D umfassen beispielsweise voneinander unabhängig eine oder mehrere Aminosäuren, organische aliphatische Reste, insbesondere gesättigte oder ungesättigte Fettsäurereste, oder auch Lipopolysaccharide.

Stehen A und D für Aminosäurereste, so ergibt sich die Anzahl der amino- und carboxyterminalen Aminosäuren für die Sequenz nach Anspruch 1 nach folgender Maßgabe:
0 bis 23 Aminosäuren im aminoterminalen Rest A, und
0 oder bis maximal (23 - Anzahl der Aminosäuren im aminoterminalen Rest A) Aminosäuren im carboxyterminalen Rest D.

Für ein Peptid mit der carboxyterminal um zwei Aminosäuren erweiterten Kernsequenz entsprechend Anspruch 10 ergibt sich die Anzahl der amino- und carboxyterminalen Aminosäuren nach folgender Maßgabe:
0 bis 21 Aminosäuren im aminoterminalen Rest A, und
0 oder bis maximal (21 - Anzahl der Aminosäuren im aminoterminalen Rest A) Aminosäuren im carboxyterminalen Rest D.

Unter der Bezeichnung Peptid im Sinne der Erfindung werden auch solche Aminosäureketten verstanden, die N-terminal und C-terminal Substitutionen aufweisen. Zu den Substitutionen können beispielsweise zählen:
Acetylierung,
Veresterung mit Fettsäuren,
Konjugation mit Zuckerresten,
Alkylierungen mit C₁- bis C₁₂-Alkyl- oder Alkenylresten.

Das erfindungsgemäße Peptid der vorstehend definierten Art umfaßt bevorzugt 7 bzw. 9 bis 20, insbesondere 8 bzw. 9 bis 16 Aminosäuren.

Vorzugsweise umfaßt das Peptid die folgende Sequenz: A_m-Ala-Leu-Asn-Glu-Asp-Leu-Ser-Ser-Trp-Thr-Ala-Ala-Asp-Thr-D_n, wobei A und D für eine oder mehrere Aminosäuren stehen und m und n die Anzahl der Aminosäuren nach folgender Maßgabe angibt: m = 0 bis 16 und n = 0 oder bis maximal (16-m).

Es konnte ein Peptid identifiziert werden, das von keinem retinalen Protein abstammt, aber hinsichtlich seiner therapeutischen Potenz deutlich wirksamer ist als das zuvor beschriebene Derivat des retinalen S-Antigens. Dieses erfindungsgemäße Peptid von 14 Aminosäuren mit der Sequenz Ala-Leu-Asn-Glu-Asp-Leu-Ser-Ser-Trp-Thr-Ala-Ala-Asp-Thr ist bei oraler Verabreichung für die Unterdrückung der Uveitis am wirksamsten. Dieses Peptid ist bislang in keinerlei funktionellen Zusammenhang mit der Uveitis gebracht worden, weder hinsichtlich der Krankheitsinduktion, noch der Therapie.

Eine weitere Besonderheit dieses Peptids besteht darin, daß es nach oraler Gabe sowohl die S-Antigen-spezifische Immunantwort unterdrückt als auch die Reaktion auf das proteinchemisch nicht verwandte IRBP verringert.

Gegenstand der Erfindung ist auch die Verwendung eines erfindungsgemäßen Peptids zur Herstellung von Mitteln zur Behandlung von Autoimmunerkrankungen, und insbesondere zur Behandlung der Uveitis.

Weiterer Gegenstand der Erfindung ist ferner die Verwendung eines erfindungsgemäßen Peptids zur Herstellung eines Mittels zur Immunisierung gegen das retinale S-Antigen, gegen das interphotorezeptorretinolbindende Protein (IRBP) und/oder gegen humane Leukozytenantigene, die die Sequenz Ala-Leu-Asn-Glu-Asp-Leu-Ser-Ser-Trp-Thr-Ala-Ala-Asp-Thr enthalten.

Die vorstehend genannten, erfindungsgemäß hergestellten Mittel können als Lösung, Suspension oder Komprimat verabreicht werden. In diesem Falle sind dann neben der Wirksubstanz übliche Hilfs- und Trägerstoffe enthalten.

Infolge der Verabreichung des Medikaments kann dann auf oralem Wege eine Toleranz induziert werden. Das Peptid sollte in einer Tagesdosis von 10 µg bis 10 mg pro Kilogramm Körpergewicht verabreicht werden.

Die Wirkung der erfindungsgemäßen Peptide kann eine regulierende Wirkung auf das Immunsystem aufgrund der Kreuzreaktivität mit anderen Selbstantigenen sein. Dementsprechend ist eine Eignung der erfindungsgemäßen Peptide zur Therapie einer Vielzahl von Autoimmunerkrankungen zu erwarten.

Die Reaktion des Immunsystems ist in diesem Zusammenhang nicht auf Blutlymphozyten begrenzt. Die humorale Immunantwort (Antikörper), Makrophagen und weitere Zellen des Immunsystems können ebenfalls mögliche Ziele der erfindungsgemäßen Peptide darstellen.

Der Nachweis des therapeutischen Potentials wurde im Tierversuch erbracht. Gruppen von 8-16 Lewis-Ratten (Alter 6-8 Wochen, Gewicht ungefähr 200 g) wurden mit dem Peptid B27PD, dem entsprechenden retinalen Protein (retinales S-Antigen (S-Ag) bzw. IRBP), einem Peptid aus dem retinalen S-Antigen (verkürztes Äquivalent zu P35: PDSAg), einem nicht-relevanten Kontrollprotein (Ovalbumin aus dem Hühnerei (OVA)), einem nicht-relevanten Kontrollpeptid (B7PD) oder physiologischer Salzlösung (PBS) gefüttert.

Die Aminosäuresequenzen der vorstehend genannten Peptide B27PD, PDSAg sowie B7PD waren dabei, wie folgt, wobei die Angabe der Aminosäuren im Ein-Buchstaben-Code vorgenommen ist:

B27PD:    A-L-N-E-D-L-S-S-W-T-A-A-D-T
PDSAg:    F-L-G-E-L-T-S-S-E-V-A-T-E-V
B7PD:     A-L-N-E-D-L-R-S-W-T-A-A-D-T.

Von diesen Peptiden sind B27PD sowie PDSAg uveitogen wirksam, d.h. nach einer Immunisierung von Objekten mit den Peptiden erkranken diese an Uveitis. Im Gegensatz dazu besitzt B7PD keine uveitogene Wirksamkeit.

Die Peptide wurden in Dosen von je 200 µg, die Proteine in 1 mg-Dosen, gelöst in PBS, jeden zweiten Tag per os verabreicht. Zwei Tage nach der dritten und letzten oralen Gabe der Proteine bzw. Peptide wurde die Uveitis durch Immunisierung mit retinalen Antigenen (S-Antigen bzw. IRBP, 35 bzw. 20 µg pro Tier, emulgiert mit Adjuvans), von denen man sicher weiß, daß sie im Rattenmodell Uveitis verursachen, ausgelöst.

Bei den nicht oral tolerisierten Kontrolltieren trat die Erkrankung 10 bis 14 Tage nach der Immunisierung auf, die Versuche wurden aus Gründen des Tierschutzgesetzes drei bis vier Tage, nachdem die letzten Tiere der positiven Kontrollgruppen erkrankten, beendet.

Der schützende Effekt der oral verabreichten Antigene zeigt sich an klinisch abgeschwächtem Krankheitsverlauf oder völlig fehlender klinischer Symptomatik, die durch histologische Untersuchungen bestätigt wurden. Die histologische Bewertung umfaßt eine Graduierung der Entzündungszeichen im Gewebe und der Zerstörung der Netzhaut, die klinische Graduierung bezieht sich auf von außen sichtbare Entzündungszeichen an der Bindehaut und im vorderen Augenabschnitt, die nicht zwingend mit dem Sehvermögen korreliert sind.

Die Ergebnisse, welche anhand der vorstehend beschriebenen, im Tiermodell durchgeführten Versuche erhalten wurden, werden in den folgenden Figuren veranschaulicht.

Fig. 1 gibt die Intensität einer S-Antigen-induzierten Uveitis nach oraler Verabreichung verschiedener Antigene (orale Toleranz) wieder.

Fig. 2 zeigt die Intensität einer IRBP-induzierten Uveitis nach oraler Verabreichung verschiedener Antigene (orale Toleranz).

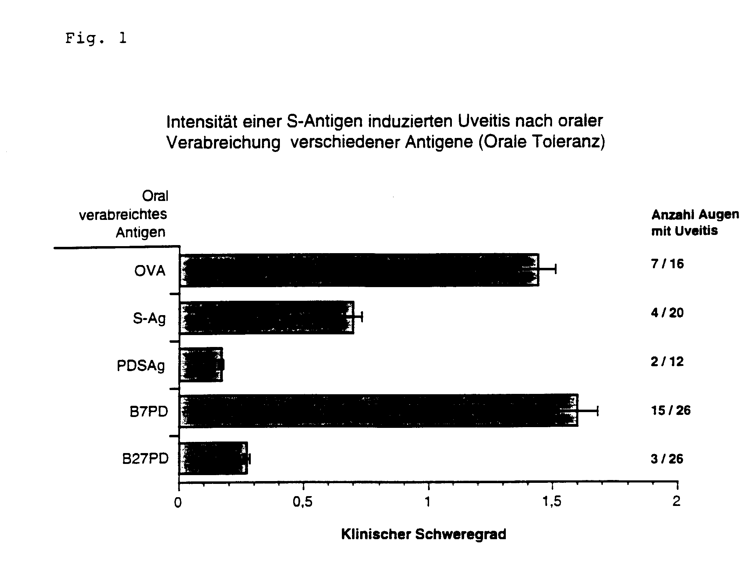

Fig. 1 zeigt, daß im Falle einer Induktion mit S-Antigen verglichen mit der Gruppe, die mit Kontrollprotein (Ovalbumin) gefüttert wurde, die klinischen Symptome durch orale Verabreichung von B27PD auf 16% verringert wurden (PDSAg: 9%, S-Ag: 41%).

Eine zusätzliche histologische Auswertung ergab, daß die Uveitis in der mit B27PD gefütterten Tiergruppe im Vergleich zur mit Ovalbumin gefütterten Kontrollgruppe histologisch um 57%, nach S-Antigen-Fütterung ebenfalls um 57% sowie nach Fütterung mit S-Antigen-Peptid nur um 14% verringert war.

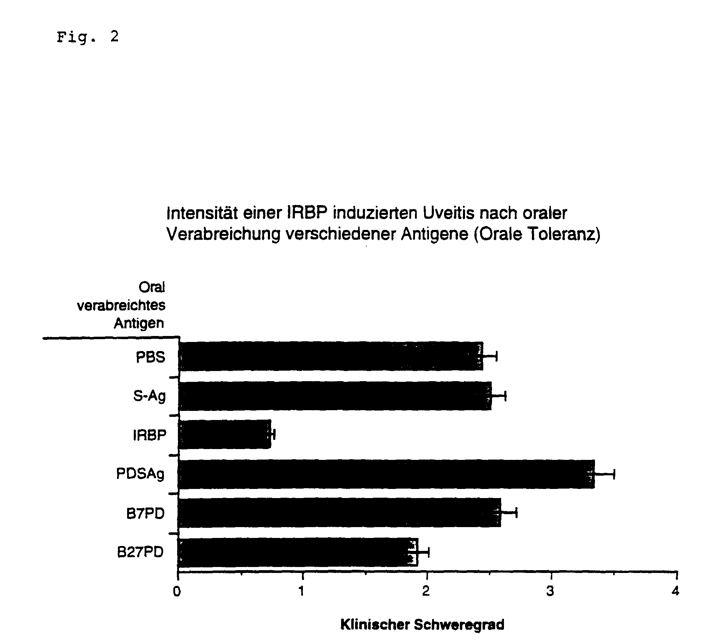

Fig. 2 zeigt, daß im Falle einer Induktion mit IRBP PBS, S-Antigen sowie auch das auf S-Antigen wirksame PDSAg, keine therapeutische Wirksamkeit besitzen. Die klinische Symptomatik kann jedoch bei B27PD-Fütterung auf 75% des bei PBS-Fütterung erhaltenen Maximalwerts reduziert werden. Nach Fütterung mit IRBP selbst wird der klinische Grad der Uveitis auf 25% gesenkt.

Es konnte gezeigt werden, daß bei diesem Peptid B27PD ohne wesentlichen Wirkungsverlust verschiedene Änderungen vorgenommen werden. Diese Änderungen umfassen unter anderen:
1) Das Peptid kann sowohl am N-terminalen als auch am C-terminalen Ende verlängert werden.
2) Das Peptid kann sowohl am N-terminalen als auch am C-terminalen Ende um einige (bis zu 6) Aminosäuren verkürzt werden.

## Patentansprüche

1. Verwendung eines Peptids aus 7 bis 30 Aminosäuren mit der Sequenz A-Leu-B-Ser-C-Thr-Ala-Ala-D, wobei B und C je für eine Aminosäure und A und D je für monomere und polymere organische Endgruppen stehen zur Herstellung eines Mittels zur oralen Verabreichung bei der Behandlung von Autoimmunerkrankungen; ausgenommen ein Peptid (B7PD), das die Sequenz Ala-Leu-Asn-Glu-Asp-Leu-Arg-Ser-Trp-Thr-Ala-Ala-Asp-Thr umfaßt.

2. Verwendung nach Anspruch 1, wobei in dem Peptid B Serin oder Threonin bedeutet.

3. Verwendung nach einem der Ansprüche 1 oder 2, wobei in dem Peptid C Threonin, Tryptophan oder Serin bedeutet.

4. Verwendung nach einem der Ansprüche 1 bis 3, wobei in dem Peptid die monomeren oder polymeren organischen Endgruppen A und B voneinander unabhängig eine oder mehrere Aminosäuren, organische aliphatische Reste oder Lipopolysaccharide umfassen können.

5. Verwendung nach einem der Ansprüche 1 bis 4, wobei das Peptid 7 bis 20 Aminosäuren umfaßt.

6. Verwendung nach Anspruch 5, wobei das Peptid 8 bis 16 Aminosäuren umfaßt.

7. Verwendung eines Peptids wie in einem der vorstehenden Ansprüche 1-6 diefiniert zur Herstellung eines Mittels zur Immunisierung gegen das retinale S-Antigen, zur Immunisierung gegen das interphotorezeptorretinolbindende Protein (IRBP) oder zur Immunisierung gegen humane Leukozytenantigene, wobei das retinale S-Antigen, IRBP oder humane Leukozytenantigen die Sequenz Ala-Leu-Asn-Glu-Asp-Leu-Ser-Ser-Trp-Thr-Ala-Ala-Asp-Thr enthält.

8. Peptid mit der Sequenz 
Aₘ-Ala-Leu-Asn-Glu-Asp-Leu-Ser-Ser-Trp-Thr-Ala-Ala-Asp-
Thr-Dₙ,
 wobei A und D für eine oder mehrere Aminosäuren stehen und m und n die Anzahl der Aminosäuren nach folgender Maßgabe angibt: m = 0 bis 16 und n = 0 oder bis maximal (16-m).

9. Peptid aus 9 bis 30 Aminosäuren mit der Sequenz A-Leu-B-Ser-C-Thr-Ala-Ala-Asp-Glu-D, wobei B und C jeweils für eine Aminosäure stehen und A und D für monomere oder polymere organische Endgruppen stehen.

10. Peptid nach Ansrpuch 9, in welchem B Serin oder Threonin bedeutet.

11. Peptid nach Anspruch 8 oder 9, in welchem C Threonin, Tryptophan oder Serin bedeutet.

12. Peptid nach einem der Ansprüche 9 bis 11, in welchem die monomeren oder polymeren organischen Endgruppen A und D voneinander unabhängig eine oder mehrere Aminosäuren, organische aliphatische Reste oder Lipopolysaccharide umfassen können.

13. Peptid nach Anspruch 12, in welchem A und/oder D ein gesättigter oder ungesättigter Fettsäurerest ist.

14. Peptid nach einem der Ansprüche 9 bis 13, umfassend 9 bis 20 Aminosäuren.

15. Peptid nach Anspruch 14, umfassend 9 bis 16 Aminosäuren.

## Claims

1. Use of a peptide consisting of 7 to 30 amino acids with the sequence A-Leu-B-Ser-C-Thr-Ala-Ala-D, wherein B and C each represent an amino acid and A and D each represent monomeric and polymeric end groups, for the preparation of a composition for oral administration for the treatment of autoimmune diseases; with the exception of a peptide (B7PD) having the sequence

Ala-Leu-Asn-Glu-Asp-Leu-Arg-Ser-Trp-Thr-Ala-Ala-Asp-Thr.

2. Use according to claim 1, wherein, in the peptide, B is serine or threonine.

3. Use according to any one of claims 1 or 2, wherein, in the peptide, C is threonine, tryptophane or serine.

4. Use according to any one of claims 1 to 3, wherein, in the peptide, the monomeric or polymeric organic end groups A and B may independently comprise one or more amino acids, organic aliphatic residues or lipopolysaccharides.

5. Use according to any one of claims 1 to 4, wherein the peptide comprises from 7 to 20 amino acids.

6. Use according to claim 5, wherein the peptide comprises from 8 to 16 amino acids.

7. Use of a peptide as defined in any one of the preceeding claims 1 - 6 for the preparation of a composition for immunization against retinal S-antigen, for immunization against interphotoreceptorretinolbinding protein (IRBP) or for immunization against human leukocyte antigens, wherein the retinal S-antigen, IRBP or human leukocyte antigen contains the sequence

Ala-Leu-Asn-Glu-Asp-Leu-Ser-Ser-Trp-Thr-Ala-Ala-Asp-Thr.

8. Peptide having the sequence A_m-Ala-Leu-Asn-Glu-Asp-Leu-Ser-Ser-Trp-Thr-Ala-Ala-Asp-Thr-D_n, wherein A and D represent one or more amino acids and m and n show the number of amino acids according to the following rule: m = 0 to 16 and n = 0 or up to maximal (16-m).

9. Peptide consisting of 9 to 30 amino acids with the sequence A-Leu-B-Ser-C-Thr-Ala-Ala-Asp-Glu-D, wherein B and C each represent an amino acid and A and D represent monomeric or polymeric organic end groups.

10. Peptide according to claim 9, wherein B is serine or threonine.

11. Peptide according to claim 8 or 9, wherein C is threonine, tryptophane or serine.

12. Peptide according to any one of claims 9 to 11, wherein the monomeric or polymeric organic end groups A and D may independently comprise one or more amino acids, organic aliphatic residues or lipopolysaccharides.

13. Peptide according to claim 12, wherein A and/or D is a saturated or unsaturated fatty acid residue.

14. Peptide according to any one of claims 9 to 13 which comprises from 9 to 20 amino acids.

15. Peptide according to claim 14 which comprises from 9 to 16 amino acids.

## Revendications

1. Utilisation d'un peptide de 7 à 30 acides aminés de séquence A-Leu-B-Ser-C-Thr-Ala-Ala-D où B et C représentent chacun un acide aminé et A et D représentent chacun des groupes terminaux organiques monomères et polymères pour la préparation d'un agent pour l'administration orale lors du traitement de maladies auto-immunes ; à l'exception d'un peptide (B7PD) qui comprend la séquence

Ala-Leu-Asn-Glu-Asp-Leu-Arg-Ser-Trp-Thr-Ala-Ala-Asp-Thr.

2. Utilisation selon la revendication 1, où B représente la sérine ou la thréonine dans le peptide.

3. Utilisation selon l'une des revendications 1 et 2, où C représente la thréonine, la tryptophane ou la sérine dans le peptide.

4. Utilisation selon l'une des revendications 1 à 3 où, dans le peptide, les groupes terminaux organiques monomères ou polymères A et B peuvent comprendre indépendamment l'un de l'autre un ou plusieurs acides aminés, restes aliphatiques organiques ou lipopolysaccharides.

5. Utilisation selon l'une des revendications 1 à 4, où le peptide comprend 7 à 20 acides aminés.

6. Utilisation selon la revendication 5, où le peptide comprend 8 à 16 acides aminés.

7. Utilisation d'un peptide tel que défini dans l'une des revendications 1 à 6 précédentes pour la production d'un agent pour l'immunisation contre l'antigène S rétinal, pour l'immunisation contre la protéine fixant le rétinol d'interphotorécepteur (IRBP) ou pour l'immunisation contre des antigènes leucocytaires humains, où l'antigène S rétinal, l'IRBP ou l'antigène leucocytaire humain contient la séquence

Ala-Leu-Asn-Glu-Asp-Leu-Ser-Ser-Trp-Thr-Ala-Ala-Asp-Thr

8. Peptide de séquence A_m-Ala-Leu-Asn-Glu-Asp-Leu-Ser-Ser-Trp-Thr-Ala-Ala-Asp-Thr-D_n où A et D représentent un ou plusieurs acides aminés et m et n représentent le nombre des acides aminés selon la condition suivante : m = 0 à 16 et n = 0 ou au maximum (16-m).

9. Peptide de 9 à 30 acides aminés de séquence A-Leu-B-Ser-C-Thr-Ala-Ala-Asp-Glu-D où B et C représentent chacun un acide aminé et A et D représentent des groupes terminaux organiques monomères ou polymères.

10. Peptide selon la revendication 9, dans lequel B représente la sérine ou la thréonine.

11. Peptide selon la revendication 8 ou 9, dans lequel C représente la thréonine, le tryptophane ou la sérine.

12. Peptide selon l'une des revendications 9 à 11, dans lequel les groupes terminaux organiques monomères ou polymères A et D peuvent comprendre indépendamment l'un de l'autre un ou plusieurs acides aminés, restes aliphatiques organiques ou lipopolysaccharides.

13. Peptide selon la revendication 12 dans lequel A et/ou D est un reste d'acide gras saturé ou insaturé.

14. Peptide selon l'une des revendications 9 à 13, comprenant 9 à 20 acides amines.

15. Peptide selon la revendication 14, comprenant 9 à 16 acides aminés.
